# EUROPEAN PATENT APPLICATION

(11) **EP 3 779 857 A1**
(43) Date of publication of application: **17.02.2021**
(21) Application number: 19785594.3
(22) Date of filing: 28.01.2019
(51) Int. Cl.: G06Q 50/10, G06F 16/00

(54) **INFORMATION PROCESSING DEVICE AND INFORMATION PROCESSING METHOD**

(30) Priority: 12.04.2018 JP 2018077088
(71) Applicant: Sony Corporation, 108-0075 Tokyo (JP)
(72) Inventor: OZAKI, Satoshi, Tokyo 108-0075 (JP)
(74) Representative: 2SPL Patentanwälte PartG mbB
(86) International application number: PCT/JP2019/002642
(87) International publication number: WO 2019/198302

(57) **Abstract**

[Problem]

To realize the presentation of information relating to health and safety which is of a higher value to a user.

[Solution]

The present disclosure provides an information processing device including an output control unit that controls notification of risk information pertaining to the health and safety of a user; and an extraction unit that, on the basis of a situation of the user, dynamically filters the risk information, which is collected on the basis of a range of actions of the user. The output control unit notifies the user of the risk information which has been filtered by the extraction unit.

## Description

### Field

The present disclosure relates to an information processing device and an information processing method.

### Background

In recent years, a great deal of technology that provides various information in response to user states and actions has been developed. For example, Patent Literature 1 discloses technology that detects or estimates a user destination and delivers neighborhood information pertaining to the destination.

### Citation List

### Patent Literature

Patent Literature 1: JP 2013-83596 A

### Summary

### Technical Problem

Further, possible information that a user needs to be provided with includes, for example, information relating to health and safety. Meanwhile, the priority level of information relating to health and safety is expected to change dynamically on the basis of different situations. Hence, in the presentation of information pertaining to health and safety, the information which is to be presented is desirably selected in view of various factors in addition to the destination of the user.

Therefore, the present disclosure proposes a new and improved information processing device and information processing method that are capable of realizing the presentation of information relating to health and safety which is of a higher value to a user.

### Solution to Problem

According to the present disclosure, an information processing device is provided that includes: an output control unit that controls notification of risk information pertaining to health and safety of a user; and an extraction unit that, on a basis of a situation of the user, dynamically filters the risk information, which is collected on a basis of a range of actions of the user, wherein the output control unit notifies the user of the risk information which has been filtered by the extraction unit.

Moreover, according to the present disclosure, an information processing method is provided that includes, by a processor, controlling notification of risk information pertaining to health and safety of a user; and dynamically filtering, on a basis of a situation of the user, the risk information, which is collected on a basis of a range of actions of the user, wherein the controlling further includes notifying the user of the filtered risk information.

### Advantageous Effects of Invention

According to the present disclosure as described hereinabove, it is possible to realize the presentation of information relating to health and safety which is of a higher value to a user.

Note that the present disclosure is not necessarily limited to or by the above-described advantageous effect and may afford any of the advantageous effects illustrated in this specification together with the above-described advantageous effect or in place of the above-described advantageous effect, or other advantageous effects that can be ascertained from this specification.

### Brief Description of Drawings

FIG. 1 is a diagram serving to illustrate an overview of an embodiment of the present disclosure.
FIG. 2 is a block diagram illustrating a configuration example of an information processing system according to the same embodiment.
FIG. 3 is a block diagram illustrating a function configuration example of an information processing terminal according to the same embodiment.
FIG. 4 is a block diagram illustrating a function configuration example of an information processing server according to the same embodiment.
FIG. 5 is a diagram serving to illustrate the collection of risk information on the basis of a profile according to the same embodiment.
FIG. 6A is a diagram serving to illustrate the acquisition of epidemic information in transportation according to the same embodiment.
FIG. 6B is a diagram serving to illustrate the acquisition of epidemic information in transportation according to the same embodiment.
FIG. 7 is a diagram serving to illustrate the collection of risk information on the basis of schedule information according to the same embodiment.
FIG. 8 is an example of epidemic information about an infectious disease according to the same embodiment.
FIG. 9 is a diagram serving to illustrate filtering of epidemic information on the basis of the state of health of a user according to the same embodiment.
FIG. 10 is a diagram serving to illustrate filtering of epidemic information on the basis of the state of health of the user according to the same embodiment.
FIG. 11 is a diagram serving to illustrate output control on the basis of user attributes according to the same embodiment.
FIG. 12 is a diagram serving to illustrate an example of relevant parties who are physically sharing a space with the user according to the same embodiment.
FIG. 13A is a diagram serving to illustrate control of epidemic information notification in a remote location according to the same embodiment.
FIG. 13B is a diagram serving to illustrate control of epidemic information notification in a remote location according to the same embodiment.
FIG. 14 is a diagram illustrating an example of epidemic information notification accompanied by visual information according to the same embodiment.
FIG. 15 is a flowchart illustrating the flow of epidemic information collection by an information processing server according to the same embodiment.
FIG. 16 is a flowchart illustrating the flow of control of epidemic information notification by an information processing server according to the same embodiment.
FIG. 17 is a flowchart illustrating the flow of state of health acquisition according to the same embodiment.
FIG. 18 is a diagram illustrating a hardware configuration example according to an embodiment of the present disclosure.

### Description of Embodiments

A preferred embodiment of the present disclosure will be described in detail hereinbelow with reference to the accompanying drawings. Note that redundant descriptions will be omitted from the present specification and the drawings by assigning the same reference signs to components having substantially the same function configuration.

Note that the description will be made in the following order.
1. Embodiment
1.1. Overview
1.2. System configuration example
1.3. Function configuration example of information processing terminal 10
1.4. Function configuration example of information processing server 20
1.5. Function details
1.6. Operation flow
2. Hardware configuration example
3. Summary

### <1. Embodiment>

### <<1.1. Overview>>

First, an overview of an embodiment of the present disclosure will be described. In recent years, through the development of information processing technology, users have come to be able to easily collect various information via the internet and the like. Such information includes, for example, risk information pertaining to health and safety.

By using a personal computer (PC) or a smartphone, or the like, to collect the foregoing risk information, which is delivered by a country or local government or by a company or individual, or similar, for example, a user is able to avoid or prevent a corresponding risk.

Here, the foregoing risk information pertaining to health and safety includes, for example, epidemic information about an infectious disease. By collecting epidemic information about an infectious disease which is prevalent in the vicinity of their home, for example, the user is able to prevent the infectious disease or prepare in advance for treatment in the event of infection.

However, locations where the user is likely to contract an infectious disease are not limited to the vicinity of the home of the user, there being a great many areas such as, for example, the workplace (or place of study) of the user, their route to work (or route to their place of study) or trip destination. Furthermore, it is also to be expected that the user may, for example, contract an infectious disease from companions performing the same action or from visitors visiting the user, and so forth. Hence, there is a huge amount of work and limits involved when a user collects, by themselves, epidemic information about an infectious disease for which there is risk of infection.

Furthermore, in cases where there is information about an infectious disease which has begun to circulate and which is highly correlated with the state of health of the user, it is normal for the user to want to quickly obtain this information and undergo treatment such as a medical examination. However, it is exceedingly difficult for the user to always check the information and it is sometimes also difficult for the user to correctly ascertain their own state of health.

The technical idea of the present disclosure was conceived in view of the foregoing points and makes it possible, in the presentation of information pertaining to health and safety, to improve user-friendliness and provide information of a higher value to the user. Therefore, an information processing server 20 that implements the information processing method according to an embodiment of the present disclosure includes an output control unit 240 that controls notification of risk information pertaining to the health and safety of a user; and an extraction unit 230 that, on the basis of a situation of the user, dynamically filters the risk information, which is collected on the basis of a range of actions of the user. In addition, one characteristic of the output control unit 240 according to an embodiment of the present disclosure is that the user is notified of risk information that has been filtered by the extraction unit 230.

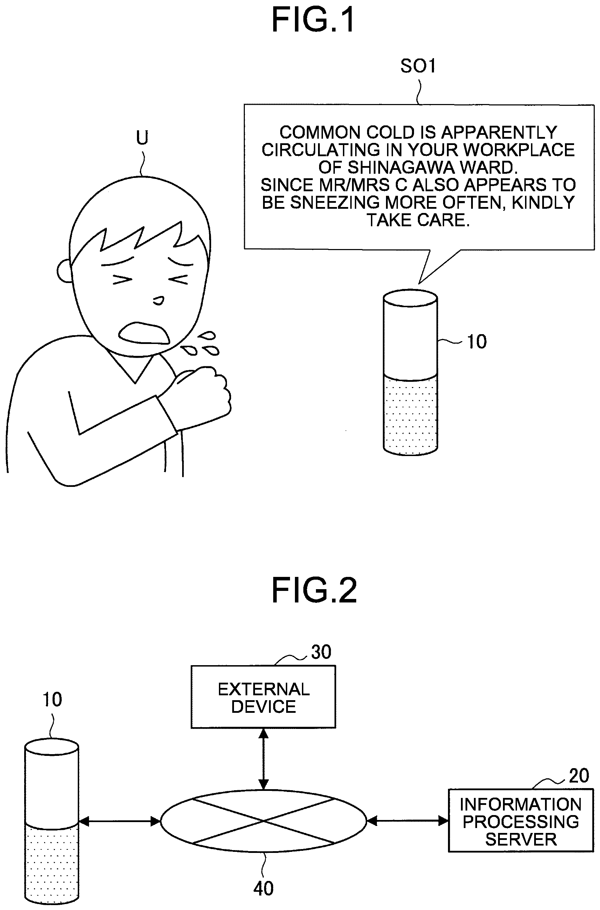

FIG. 1 is a diagram serving to illustrate an overview of the present embodiment. FIG. 1 illustrates an example of a case where the information processing server 20 according to the present embodiment performs control of the output of epidemic information about an infectious disease as an example of risk information pertaining to health and safety.

First, the information processing server 20 according to the present embodiment collects a wide range of epidemic information about an infectious disease, which is delivered by a country or local government or by a company or individual, or similar, on the basis of a range of actions acquired from the profile of a user U and from schedule information. The foregoing profile includes information such as a place of residence, a workplace (place of study), and a route to work (or route to place of study), for example.

Furthermore, the information processing server 20 according to the present embodiment dynamically filters the collected infectious disease epidemic information on the basis of the schedule information about user U and a situation such as their state of health, and notifies the information processing terminal 10 of epidemic information which is of a higher value to user U.

In the case of the example illustrated in FIG. 1, after it has been detected that the number of sneezes by user U has increased, the information processing server 20 extracts, on the basis of the workplace of user U, epidemic information about an infectious disease (the common cold) for which sneezing occurs as a symptom from among collected epidemic information, and outputs the extracted information to the information processing terminal 10 as a speech utterance SO1.

As described hereinabove, the information processing server 20 according to the present embodiment makes it possible to collect, on the basis of a range of user actions, a wide range of epidemic information about an infectious disease which the user may contract. Furthermore, the information processing server 20 according to the present embodiment is able to realize the provision of information of a higher value to the user by filtering the collected epidemic information on the basis of the state of health of the user, and so forth.

According to the foregoing function of the information processing server 20 according to the present embodiment, the user is able to ascertain information about an infectious disease that is circulating in their own area without consciously collecting information, and is able to prevent an infectious disease, quickly ascertain the possibility of infection, and deal with the infectious disease by consulting a medical institution, or the like.

An overview of the present embodiment has been described hereinabove. Note that although a case where the information processing server 20 according to the present embodiment controls information notification pertaining to infectious disease epidemic information is described hereinbelow as a principal example, the risk information pertaining to health and safety of the present embodiment is not limited to or by the associated example. The risk information of the present embodiment may include various information affecting the health and safety of the user. The risk information of the present embodiment includes a wide range of information such as, for example, dispersal information such as pollen or PM 2.5 information, atmospheric temperature and humidity (or various indices calculated from the atmospheric temperature and humidity and the like), and information about the occurrence of incidents or accidents.

### <<1.2. System configuration example>>

Next, a configuration example for an information processing system according to an embodiment of the present disclosure will be described. FIG. 2 is a block diagram illustrating a configuration example of the information processing system according to the present embodiment. Referring to FIG. 2, the information processing system according to the present embodiment includes an information processing terminal 10, an information processing server 20, and an external device 30. Furthermore, the information processing terminal 10 and the information processing server 20, and the information processing server 20 and the external device 30 are interconnected via a network 40 so as to enable mutual communication therebetween.

### (Information processing terminal 10)

The information processing terminal 10 according to the present embodiment is an information processing device for notifying a user of risk information pertaining to health and safety, on the basis of control by the information processing server 20. Therefore, the information processing terminal 10 according to the present embodiment has a function for outputting speech and visual information.

The information processing terminal 10 according to the present embodiment may, for example, be a PC, a portable telephone, a smartphone, a tablet, a wearable device, or an agent device of a stationary type or autonomous mobile type.

### (Information processing server 20)

The information processing server 20 according to the present embodiment is an information processing device that controls the notification of risk information by the information processing terminal 10. Furthermore, the information processing server 20 collects risk information pertaining to health and safety from the external device 30 via the network 40. Details of the functions of the information processing server 20 according to the present embodiment will be separately described subsequently.

### (External device 30)

The external device 30 according to the present embodiment is an information processing device that is installed by a country or local government or a company or individual for the purpose of delivering risk information pertaining to health and safety.

### (Network 40)

The network 40 has a function for connecting the information processing terminal 10 to the information processing server 20. The network 40 may also include a public line such as the internet, a telephone network, or a satellite communications network, and various local area networks (LAN) including Ethernet (registered trademark) and wide area networks (WAN), or the like. Furthermore, the network 40 may also include a leased line network such as an internet protocol-virtual private network (IP-VPN). In addition, the network 40 may also include a wireless communications network such as Wi-Fi (registered trademark) or Bluetooth (registered trademark).

A configuration example of the information processing system according to the present embodiment has been described above. Note that the above-described configuration described using FIG. 2 is merely an example, and the configuration of the information processing system according to the present embodiment is not limited to the example. For example, the functions of the information processing terminal 10 and the information processing server 20 according to the present embodiment may be realized by a single device. The configuration of the information processing system according to the present embodiment can be flexibly modified in accordance with specifications and operations.

### <<1.3. Function configuration example of information processing terminal 10>>

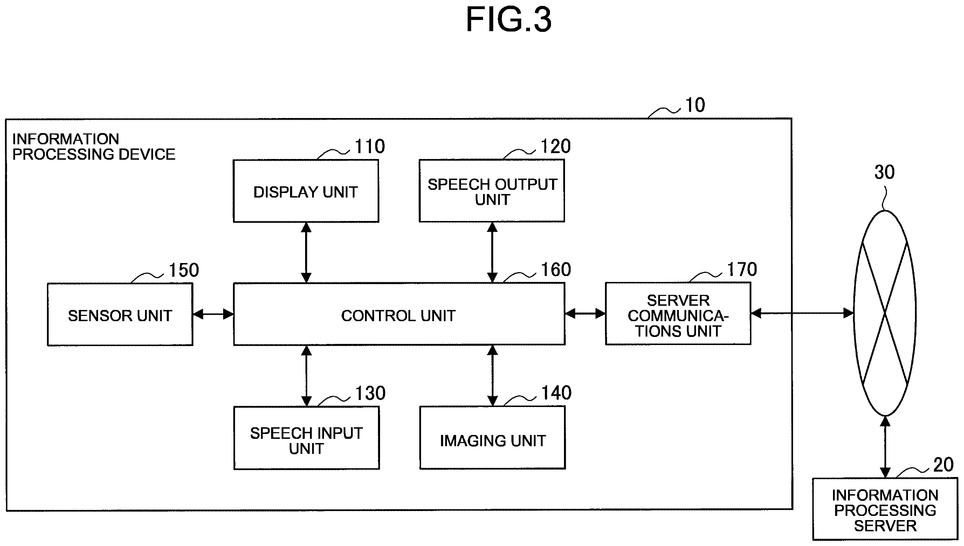

Next, a function configuration example of the information processing terminal 10 according to the present embodiment will be described. FIG. 3 is a block diagram illustrating a function configuration example of the information processing terminal 10 according to the present embodiment. Referring to FIG. 3, the information processing terminal 10 according to the present embodiment includes a display unit 110, a speech output unit 120, a speech input unit 130, an imaging unit 140, a sensor unit 150, a control unit 160, and a server communications unit 170.

### (Display unit 110)

The display unit 110 according to the present embodiment has a function for outputting visual information such as images and text. The display unit 110 according to the present embodiment outputs risk information pertaining to health and safety on the basis of control by the information processing server 20, for example.

To this end, the display unit 110 according to the present embodiment includes a display device that presents visual information. Examples of the display device described above include a liquid crystal display (LCD) device, an organic light emitting diode (OLED) device, and a touch panel, and the like. Further, the display unit 110 according to the present embodiment may use a projection function to output the visual information.

### (Speech output unit 120)

The speech output unit 120 according to the present embodiment has a function for outputting various sounds including speech. The speech output unit 120 according to the present embodiment uses speech to output risk information pertaining to health and safety on the basis of control by the information processing server 20, for example. To this end, the speech output unit 120 according to the present embodiment includes speech output devices such as a loudspeaker and an amplifier.

### (Speech input unit 130)

The speech input unit 130 according to the present embodiment has a function for collecting sound information such as user utterances and ambient sound occurring in the vicinity of the information processing terminal 10. The speech input unit 130 according to the present embodiment includes a microphone for collecting the sound information. The sound information collected by the speech input unit 130 may be used by the information processing server 20 in acquiring the state of health of the user, for example.

### (Imaging unit 140)

The imaging unit 140 according to the present embodiment has a function for capturing images of the user and the peripheral environment. The images captured by the imaging unit 140 may be used by the information processing server 20 in identifying the user and in acquiring the state of health of the user, and so forth. The imaging unit 140 according to the present embodiment includes an imaging device capable of capturing images. Note that the above images include moving images as well as still images.

### (Sensor unit 150)

The sensor unit 150 according to the present embodiment has a function for collecting various sensor information relating to the peripheral environment and to the user. The sensor information collected by the sensor unit 150 may be used by the information processing server 20 in acquiring the state of health of the user, for example. The sensor unit 150 includes various biometric sensors including an infrared sensor, or the like, for example.

### (Control unit 160)

The control unit 160 according to the present embodiment has a function for controlling the respective configurations that the information processing terminal 10 includes. The control unit 160 controls the starting and stopping of each configuration, for example. Furthermore, the control unit 160 inputs a control signal which is generated by the information processing server 20 to the display unit 110 and the speech output unit 120. Further, the control unit 160 according to the present embodiment may have the same functions as the extraction unit 230 and the output control unit 240 of the information processing server 20 described subsequently.

### (Server communications unit 170)

The server communications unit 170 according to the present embodiment has a function for communicating information with the information processing server 20 via the network 40. More specifically, the server communications unit 170 transmits, to the information processing server 20, sound information collected by the speech input unit 130, image information captured by the imaging unit 140, and sensor information collected by the sensor unit 150. Furthermore, the server communications unit 170 receives, from the information processing server 20, a control signal pertaining to the output of risk information relating to health and safety, or the like.

The function configuration example of the information processing terminal 10 according to the present embodiment has been described above. Note that the above configuration described using FIG. 3 is merely an example, and the function configuration of the information processing terminal 10 according to the present embodiment is not limited to the example. For example, the information processing terminal 10 according to the present embodiment does not necessarily include all of the configurations illustrated in FIG. 3. For example, the information processing terminal 10 may also be configured to not include the display unit 110, or the like. Furthermore, as mentioned hereinabove, the control unit 160 according to the present embodiment may have the same functions as the extraction unit 230 and output control unit 240 of the information processing server 20. The function configuration of the information processing terminal 10 according to the present embodiment can be flexibly modified in accordance with specifications and the application.

### <<1.4. Function configuration example of information processing server 20>>

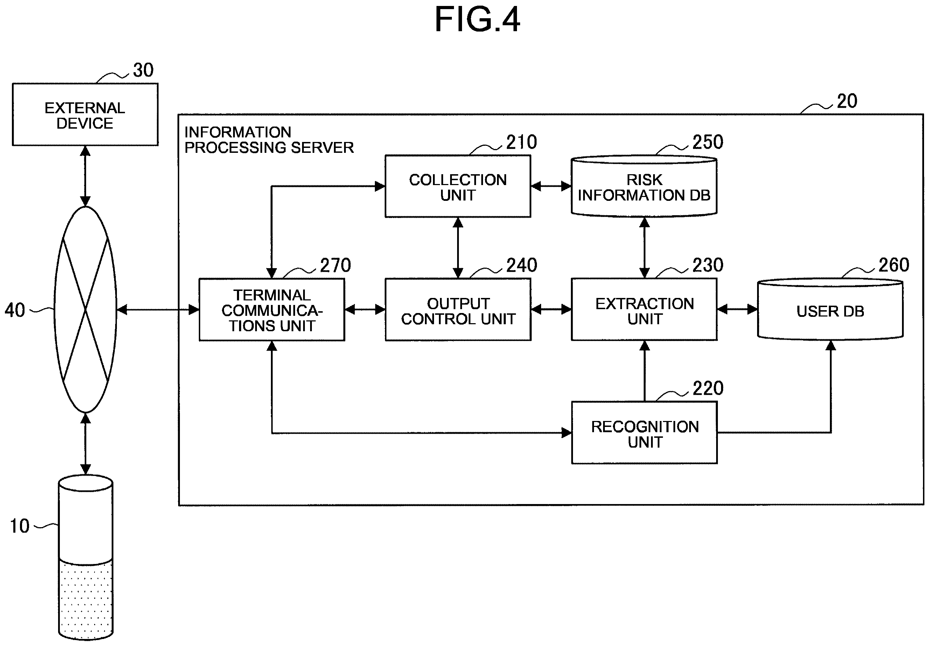

Next, a function configuration example of the information processing server 20 according to an embodiment of the present disclosure will be described. FIG. 4 is a block diagram illustrating a function configuration example of the information processing server 20 according to the present embodiment. Referring to FIG. 4, the information processing server 20 according to the present embodiment includes a collection unit 210, a recognition unit 220, an extraction unit 230, an output control unit 240, a risk information DB 250, a user DB 260, and a terminal communications unit 270.

### (Collection unit 210)

The collection unit 210 according to the present embodiment has a function for collecting, on the basis of a range of user actions, risk information, pertaining to health and safety from the external device 30 and the internet, which corresponds to the range of actions. The collection unit 210 according to the present embodiment acquires the foregoing range of actions on the basis of the profile and schedule information of the user, which will be described subsequently. The collection unit 210 stores the collected risk information in the risk information DB 250.

### (Recognition unit 220)

The recognition unit 220 according to the present embodiment has a function for identifying the user and acquiring the state of health of the user, on the basis of sound information, image information, and sensor information which have been collected by the information processing terminal 10. The recognition unit 220 is capable of recognizing the number and frequency of coughs and sneezes by the user on the basis of the sound information and image information, for example. Furthermore, the recognition unit 220 is capable of measuring the temperature and heart rate of the user on the basis of image information and sensor information which has been collected by the infrared sensor, for example.

### (Extraction unit 230)

The extraction unit 230 according to the present embodiment has a function for dynamically filtering, on the basis of the schedule and state of health of the user, risk information which has been collected by the collection unit 210 and stored in the risk information DB 250.

The extraction unit 230 according to the present embodiment may dynamically calculate the priority levels of respective risk information on the basis of the state of health of the user and execute filtering of the risk information on the basis of the priority levels, for example. More specifically, the extraction unit 230 according to the present embodiment is capable of extracting risk information of a higher value to the user by calculating as higher the priority level of the risk information which is highly correlated with the state of health of the user. Details of the functions of the extraction unit 230 according to the present embodiment will be separately described subsequently.

### (Output control unit 240)

The output control unit 240 according to the present embodiment has a function for causing the information processing terminal 10 to notify the user of risk information that has been filtered by the extraction unit 230. The output control unit 240 according to the present embodiment causes the information processing terminal 10 to output the foregoing risk information by means of speech and visual information, for example.

### (Risk information DB 250)

The risk information DB 250 according to the present embodiment is a database that stores the risk information collected by the collection unit 210.

### (User DB 260)

The user DB 260 according to the present embodiment is a database that stores various information relating to users. The user DB 260 according to the present embodiment stores user profiles, for example. Here, the foregoing profiles include, in addition to the age and gender of the user, their place of residence, workplace (place of study), and route to work (or route to their place of study), for example. Furthermore, the user DB 260 stores user schedule information. The foregoing schedule information includes, in addition to a destination and a travel route, information about their companions and visitors, and so forth.

### (Terminal communications unit 270)

The terminal communications unit 270 according to the present embodiment communicates information with the information processing terminal 10 and the external device 30 via the network 40. For example, the terminal communications unit 270 receives, from the information processing terminal 10, sound information, image information, and sensor information, and the like. Further, the terminal communications unit 270 transmits, to the information processing terminal 10, a control signal pertaining to the notification of risk information as generated by the output control unit 240. In addition, the terminal communications unit 270 receives the risk information from the external device 30 on the basis of control by the collection unit 210.

A function configuration example of the information processing server 20 according to an embodiment of the present disclosure has been described hereinabove. Note that the foregoing configuration described using FIG. 4 is merely an example, and that the function configuration of the information processing server 20 according to the present embodiment is not limited to or by such an example. For example, the configuration described above may be implemented by being distributed between a plurality of devices. In addition, as mentioned earlier, the functions of the information processing terminal 10 and the information processing server 20 may be realized by a single device. The function configuration of the information processing server 20 according to the present embodiment can be flexibly modified in accordance with specifications and the application.

### <<1.5. Function details>>

Thereafter, the functions of the information processing server 20 according to the present embodiment will be described in detail. First, the function for collecting risk information of the collection unit 210 according to the present embodiment will be described in detail.

As mentioned earlier, the collection unit 210 according to the present embodiment has a function for collecting, on the basis of a range of user actions, risk information corresponding to the range of actions from the external device 30 and the internet. At such time, the collection unit 210 according to the present embodiment acquires a range of actions on the basis of the profile of the user and collects risk information on the basis of the range of actions, for example.

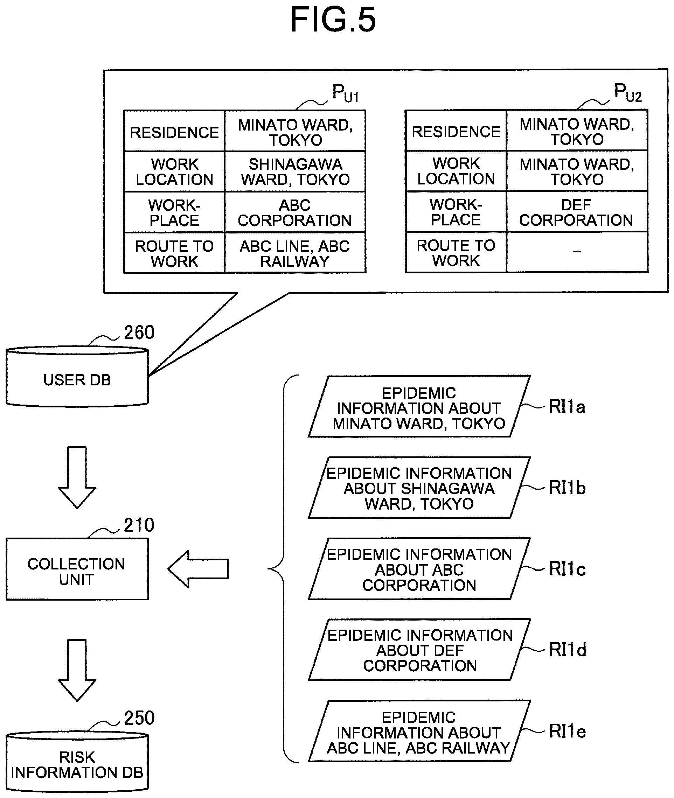

FIG. 5 is a diagram serving to illustrate the collection of risk information on the basis of a profile according to the present embodiment. FIG. 5 illustrates an example of a case where the collection unit 210 according to the present embodiment collects infectious disease epidemic information on the basis of a user profile which is stored in the user DB 260.

In the case of the example illustrated in FIG. 5, the user DB 260 stores a profile P_{U1} of a user U1 and a profile P_{U2} of a user U2. Here, user U1 and user U2 may be a couple, for example. Furthermore, profile P_{U1} and profile P_{U2} include information relating to the place of residence, work location (study location), workplace (place of study), and route to work (route to place of study) of users U1 and U2, respectively.

At such time, the collection unit 210 according to the present embodiment acquires a range of actions of user U1 and user U2 from profile P_{U1} and profile P_{U2} and collects infectious disease epidemic information corresponding to this range of actions from the external device 30 and the internet.

In the case of the example illustrated in FIG. 5, the collection unit 210 collects epidemic information RI1a to RIle on the basis of profile P_{U1} and profile P_{U2} and stores this information in the risk information DB 250.

Here, the epidemic information RI1a may be infectious disease epidemic information for Minato ward, Tokyo, which is the place of residence of users U1 and U2 and the work location of user U2. In addition, the epidemic information RI1b may be infectious disease epidemic information for Minato ward, Tokyo, which is the work location of user U1.

Furthermore, the epidemic information RI1c and RI1d may be infectious disease epidemic information for the workplaces of users U1 and U2, respectively. Further, the epidemic information RIle may be infectious disease epidemic information for a railway which user U1 uses on their route to work.

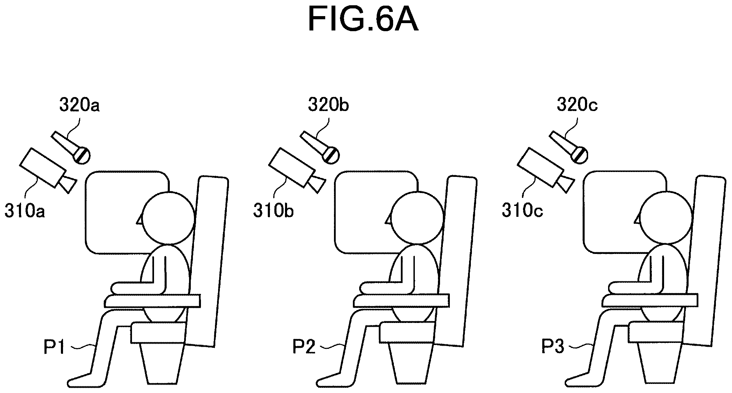

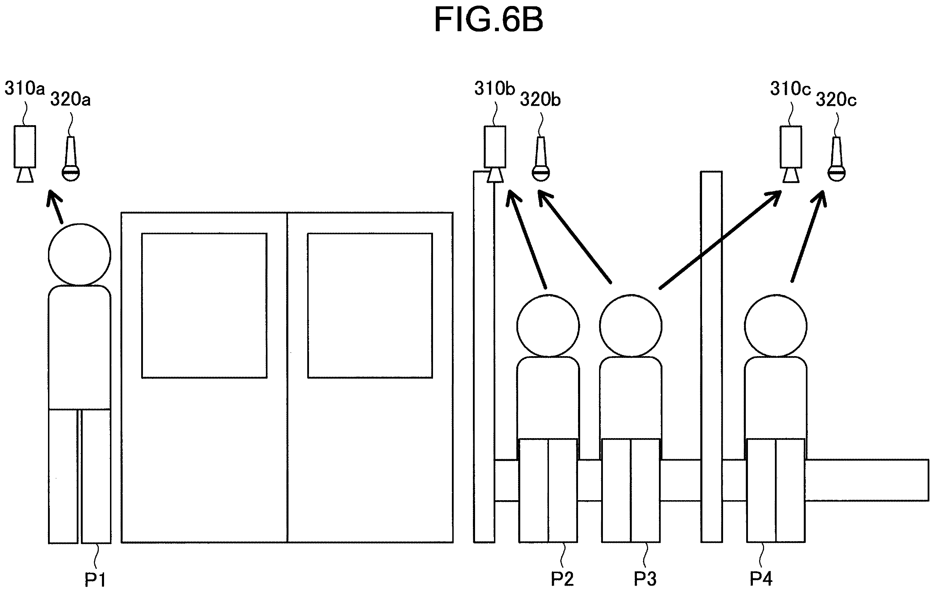

Note that infectious disease epidemic information about transportation which is used on a route to work and so forth may be acquired on the basis of information which is collected by an imaging device and a microphone, or the like, which are installed by the company providing the transportation, for example. FIGS. 6A and 6B are diagrams serving to illustrate the acquisition of epidemic information in transportation according to the present embodiment.

For example, FIG. 6A illustrates imaging devices 310a to 310c and microphones 320a to 320c which are installed in a carriage by the company providing the means of transportation such as a shinkansen. The imaging devices 310a to 310c and microphones 320a to 320c are installed for passengers P1 to P3 and are capable of collecting states of health such as coughing, sneezing, and fever for each passenger. Note that the imaging device 310 and microphone 320 may also be installed in the ratio of one to a plurality of passengers.

Further, FIG. 6B illustrates an example of the installation of imaging device 310 and microphone 320 on a conventional line, for example. In the case of the example illustrated in FIG. 6B, the imaging devices 310a to 310c and microphones 320a to 320c collect the states of health of a plurality of passengers P1 to P4.

Thus, the collection unit 210 according to the present embodiment makes it possible to automatically collect infectious disease epidemic information in various locations relevant to a user on the basis of a user profile and to markedly reduce the burden on the user to collect information.

Note that the collection unit 210 may collect epidemic information by performing crawling at regular or irregular intervals. Furthermore, the collection unit 210 is also capable of performing the collection of epidemic information in response to a profile being changed, for example.

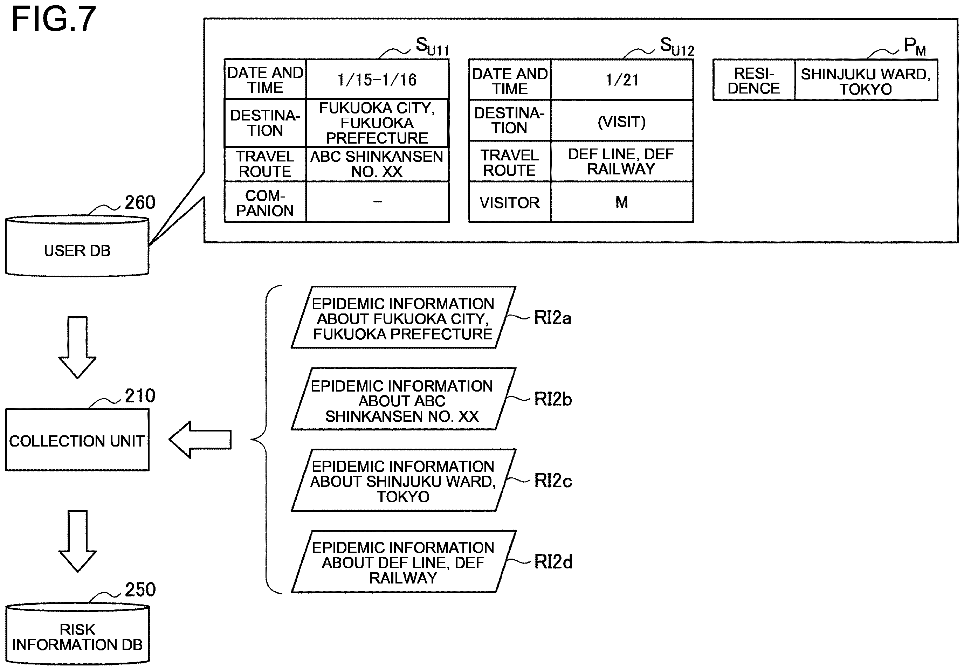

In addition, the collection unit 210 according to the present embodiment may acquire a range of actions on the basis of user schedule information and collect risk information on the basis of the range of actions. FIG. 7 is a diagram serving to illustrate the collection of risk information on the basis of schedule information according to the present embodiment. FIG. 7 illustrates an example of a case where the collection unit 210 according to the present embodiment collects infectious disease epidemic information on the basis of user schedule information which is stored in the user DB 260.

In the case of the example illustrated in FIG. 7, the user DB 260 stores two schedule information items S_{U11} and S_{U12} relating to user U1. Here, schedule information S_{U11} is information relating to a business trip schedule of user U1, and schedule information S_{U11} is information relating to a schedule in which a visitor M is coming to visit user U1.

Thus, in addition to the date and time, destination, and travel route, the schedule information according to the present embodiment may also include information about companions and visitors. In the case of the example illustrated in FIG. 7, the user DB 260 jointly stores the profile P_{M} of visitor M.

At such time, the collection unit 210 according to the present embodiment is capable of collecting infectious disease epidemic information from the external device 30 and the internet on the basis of the schedule information items S_{U11} and S_{U12} and profile P_{M}.

In the case of the example illustrated in FIG. 7, the collection unit 210 collects epidemic information RI2a to RI2d on the basis of the schedule information items S_{U11} and S_{U12} and profile P_{M} and stores this information in the risk information DB 250.

Here, the epidemic information RI2a is infectious disease epidemic information about the destination in the schedule information S_{U11}, and the epidemic information RI2b is infectious disease epidemic information about the shinkansen which corresponds to the travel route.

Furthermore, the epidemic information RI2c is infectious disease epidemic information about the place of residence of visitor M of schedule information S_{U12}, and epidemic information RI2d is infectious disease epidemic information about the railway which visitor M uses for their visit.

Thus, the collection unit 210 according to the present embodiment makes it possible to automatically collect, on the basis of user schedule information, infectious disease epidemic information relevant to the destination and travel route of the user. Further, the collection unit 210 according to the present embodiment makes it possible to collect epidemic information by taking into account not only a user profile but also the profiles of companions and visitors pertaining to a schedule, and enables the extraction of a wide range of factors that may be pathways for contracting the infectious disease.

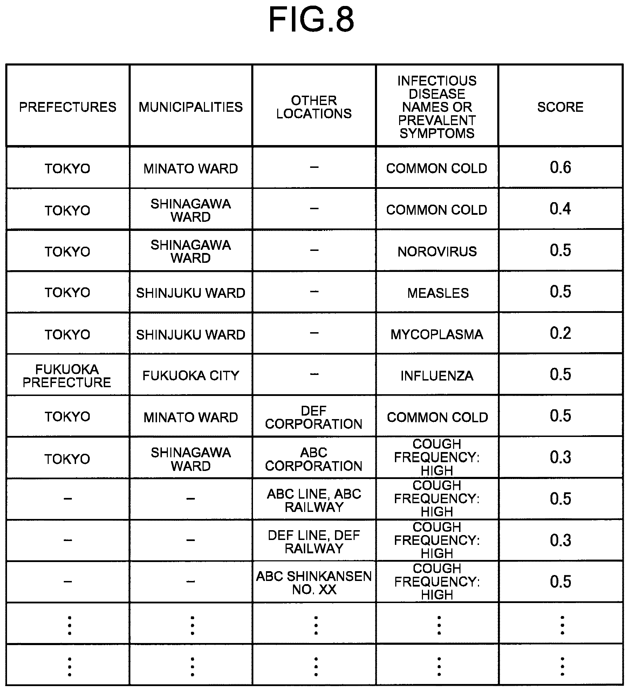

The collection, by the collection unit 210 according to the present embodiment, of epidemic information which is based on profiles and schedule information has been described hereinabove. FIG. 8 is an example of infectious disease epidemic information which is collected as described earlier and stored by the risk information DB 250.

FIG. 8 illustrates an example of epidemic information which is collected by the collection unit 210 and stored in the risk information DB 250 on the basis of the profiles and schedule information illustrated in FIGS. 5 and 7.

As illustrated in FIG. 8, the risk information DB 250 according to the present embodiment may store, for example, information relating to prefectures, municipalities, or other locations (for example, workplaces, routes to work, and so forth) where infectious diseases are circulating, content on infectious disease names and prevalent symptoms, or scores pertaining to epidemicity. Note that scores pertaining to epidemicity may be calculated on the basis of an observed increase or decrease in patients (for example, a net daily change, a net monthly change, or similar), for example.

Thus, the collection unit 210 according to the present embodiment makes it possible to collect a wide array of epidemic information relating to various locations on the basis of a range of user actions and a range of actions of relevant parties such as companions or visitors who may be factors in contracting an infectious disease, thereby enabling a significant reduction in the burden on the user to collect information.

However, if the user is notified of all the epidemic information stored in the risk information DB 250, information overload will result, and it is also assumed likely that the epidemic information which the user truly needs will be buried among other information. Therefore, the extraction unit 230 according to the present embodiment may dynamically calculate the priority levels of epidemic information on the basis of a situation of the user and may, by filtering the epidemic information on the basis of the priority levels, preferentially extract epidemic information which is of a higher value to the user.

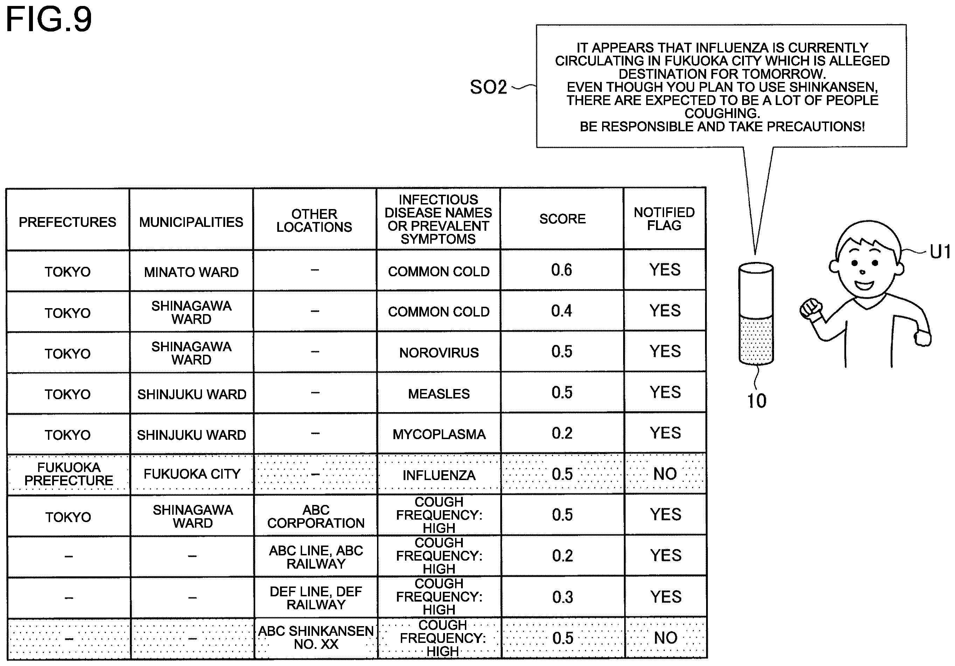

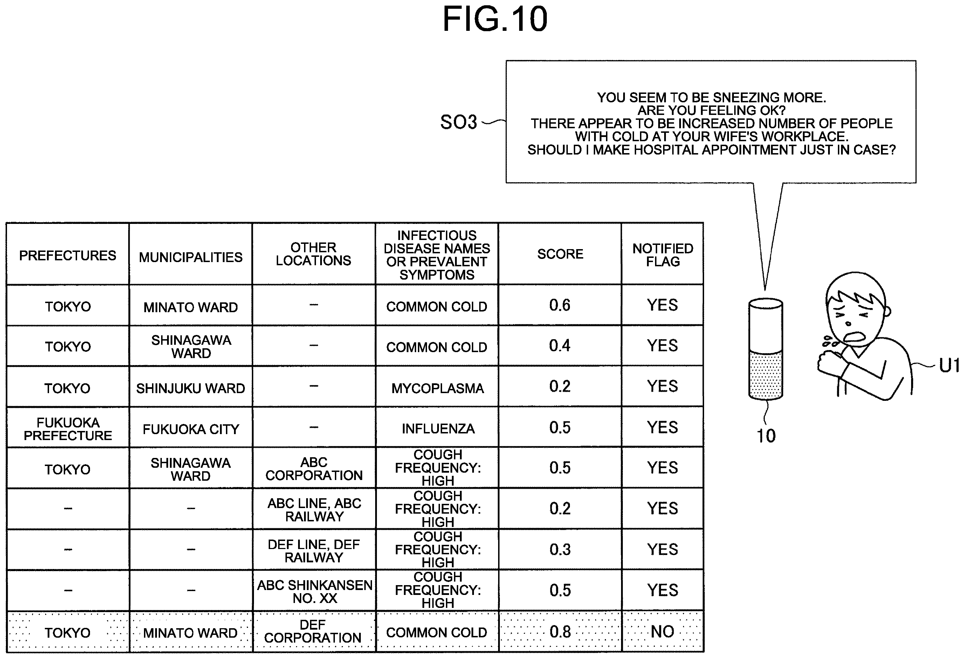

At such time, the extraction unit 230 according to the present embodiment may filter the epidemic information on the basis of the state of health of the user as recognized by the recognition unit 220, for example. FIGS. 9 and 10 are diagrams serving to illustrate filtering of epidemic information on the basis of the state of health of a user according to the present embodiment.

FIG. 9 illustrates an example of filtering of epidemic information in a case where no problems with the state of health of the user have been detected.

For example, when the state of health of user U1 is favorable, it is assumed that there is not a high probability of user U1 contracting an infectious disease from a relevant party such as user U2, who is a companion or visitor pertaining to the schedule of user U1 or who is a family member of user U1.

Therefore, in order to avoid a situation where the epidemic information extracted on the basis of the aforementioned relevant party does not provoke an information overload for user U1, the extraction unit 230 may preferentially extract epidemic information in locations which are directly visited by user U1 (their place of residence, work location, workplace, route to work, destination, and travel route), as illustrated on the left side of the drawing.

At such time, the output control unit 240 according to the present embodiment may output, to the information processing terminal 10, epidemic information not yet notified among the epidemic information extracted by the extraction unit 230 (epidemic information for which the notified flag in the drawing is NO) when the recognition unit 220 has recognized the user. Note that the foregoing notified flag may be reset at an appointed time every day, for example.

Furthermore, when the target is epidemic information which is collected on the basis of schedule information, the output control unit 240 is capable of outputting epidemic information to the information processing terminal 10 with timing which is of a higher value to the user, such as directly before the user departs for a destination, or on the preceding day.

For example, in the case of the example illustrated in FIG. 9, the output control unit 240 uses a speech utterance SO2 to output, to the information processing terminal 10, infectious disease epidemic information about a next-day destination or travel route of user U1, based on the fact that the recognition unit 220 has recognized user U1.

Thus, the extraction unit 230 and output control unit 240 according to the present embodiment make it possible to perform control so that epidemic information which is more directly correlated with the user is preferentially reported in cases where the state of health of the user is favorable.

However, FIG. 10 illustrates an example of filtering of epidemic information in a case where a problem with the state of health of the user has been detected.

In a case where a problem with the state of health of the user has been detected, it is desirable that epidemic information which is highly correlated with the state of health of the user should be preferentially extracted and that the user should be notified. Hence, in a case where a problem with the state of health of the user has been detected, the extraction unit 230 according to the present embodiment may preferentially extract, among epidemic information which has been collected on the basis of the range of actions of the user and relevant parties, epidemic information pertaining to an infectious disease for which symptoms are similar to the state of health of the user.

For example, in the case of the example illustrated in FIG. 10, the extraction unit 230 extracts epidemic information about the common cold at the workplace of user U2, who is a relevant party for user U1, as illustrated on the left side of the drawing, based on the fact that the recognition unit 220 has recognized an increase in sneezing by user U1.

In addition, the output control unit 240 uses a speech utterance SO3 to output, to the information processing terminal 10, epidemic information about the common cold at the workplace of user U2, based on the fact that the recognition unit 220 has recognized user U1.

Thus, the extraction unit 230 and output control unit 240 according to the present embodiment make it possible to realize the provision of information of a higher value to the user by performing control so that, in a case where a problem with the state of health of the user has been detected, epidemic information about an infectious disease which is similar to user symptoms is preferentially notified.

Furthermore, at such time, the output control unit 240 according to the present embodiment may ask user U1 whether a hospital appointment is necessary, as illustrated, for example. Here, when user U1 has issued an appointment instruction, the output control unit 240 may execute appointment processing by accessing a hospital appointment system or the like via the network 40.

Thus, the output control unit 240 according to the present embodiment is also capable of executing, in conjunction with control of epidemic information notification, various processing relating to the epidemic information. The output control unit 240 is capable of placing an order for a mask or some medicine and of controlling a humidifier, an air cleaner, or a ventilation fan, for example.

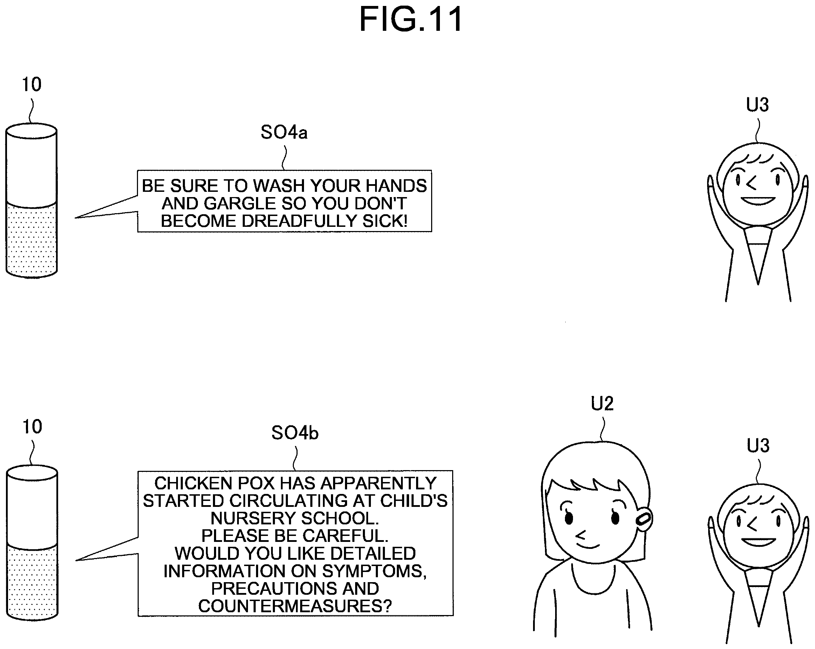

Furthermore, the output control unit 240 according to the present embodiment is also capable of dynamically modifying content which is outputted to the information processing terminal 10 on the basis of user attributes and the like which represent targets for epidemic information notification. FIG. 11 is a diagram serving to illustrate output control on the basis of user attributes according to the present embodiment.

FIG. 11 illustrates an example of a case where a user U3 who is a target for epidemic information notification is a child without sufficient knowledge regarding an infectious disease.

In this case, upon recognizing that user U3 is alone, the output control unit 240 according to the present embodiment may use a voice utterance SO4a to output, to the information processing terminal 10, precautions such as handwashing and gargling, which can be understood by user U3, without touching on the specific name and so forth of an infectious disease, as illustrated at the top of the drawing, for example.

Meanwhile, as illustrated at the bottom of the drawing, for example, upon recognizing that user U3, who is the guardian of user U2, is with user U2, the output control unit 240 may use voice utterance SO4a to output, to the information processing terminal 10, detailed epidemic information in order to enable user U2 to accurately grasp the epidemic information.

Thus, the output control unit 240 according to the present embodiment makes it possible to realize the provision of information of a higher value to the user by controlling output content on the basis of user attributes which represent epidemic information notification targets. Note that the foregoing guardian is not limited to being someone who is protecting a minor and includes a wide range of people who are responsible for the health of a user constituting a notification target, such as a care giver, for example.

In addition, the output control unit 240 according to the present embodiment may output, to the information processing terminal 10, an inquiry about whether the user would like more detailed information on the symptoms of an infectious disease, precautions, or countermeasures, as illustrated at the bottom of the drawing. Here, when the user would like more detailed information to be provided, the output control unit 240 is also capable of extracting knowledge relating to the relevant infectious disease from the internet and so forth and of providing the user with this knowledge. Thus, the output control unit 240 according to the present embodiment makes it possible to realize the presentation of information of a higher value by providing the user with additional information alongside the collected epidemic information.

The filtering and notification control of epidemic information according to the present embodiment have been described hereinabove. Note that although a case where relevant parties who may represent factors for contracting an infectious disease are family members, companions, or visitors, or the like, has been described in the foregoing as a principal example, the relevant parties according to the present embodiment are not limited to or by such an example. Relevant parties according to the present embodiment may include a variety of people who are physically sharing a space with the user constituting the notification target, for example.

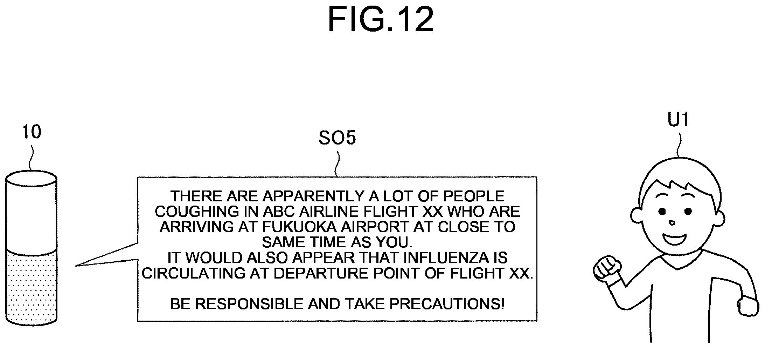

FIG. 12 is a diagram serving to illustrate an example of relevant parties who are physically sharing a space with the user according to the present embodiment. In the case illustrated in FIG. 12, the information processing server 20 regards the passengers of an aircraft arriving at an airport at nearly the same time as the aircraft which user U1 is traveling on as relevant parties and uses a speech utterance SO5 to output, to the information processing terminal 10, epidemic information which has been collected on the basis of the relevant parties.

The foregoing function of the information processing server 20 according to the present embodiment also makes it possible to effectively reduce the likelihood of a user contracting an infectious disease from a person who is sharing the same physical space at a baggage claim or at immigration, or the like, for example. Note that the foregoing physical space is not limited to being a transportation system such as an airport and may involve various spaces such as the classrooms of an educational institution, for example. Note that, in the case of classrooms and the like, there may be a temporal separation in the sharing of the physical space between the relevant parties and the user.

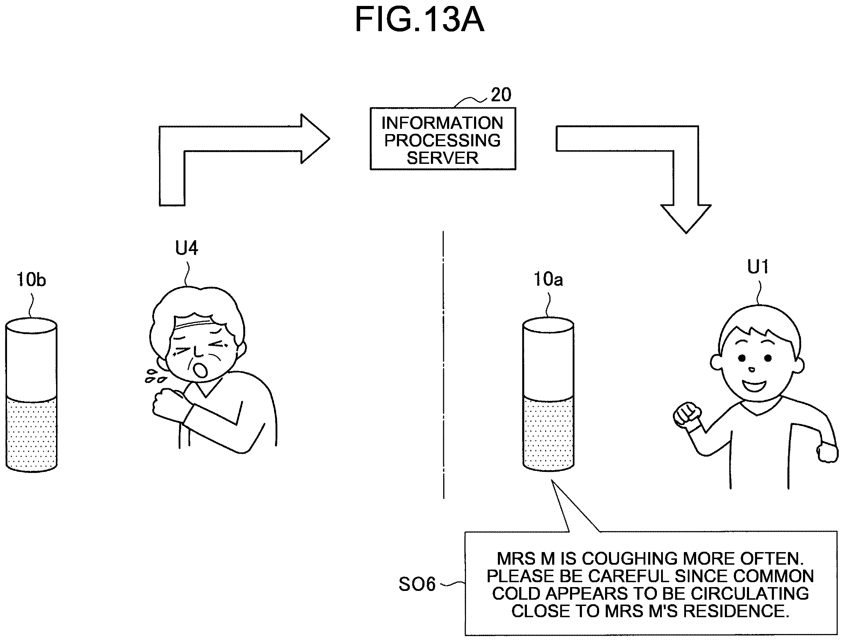

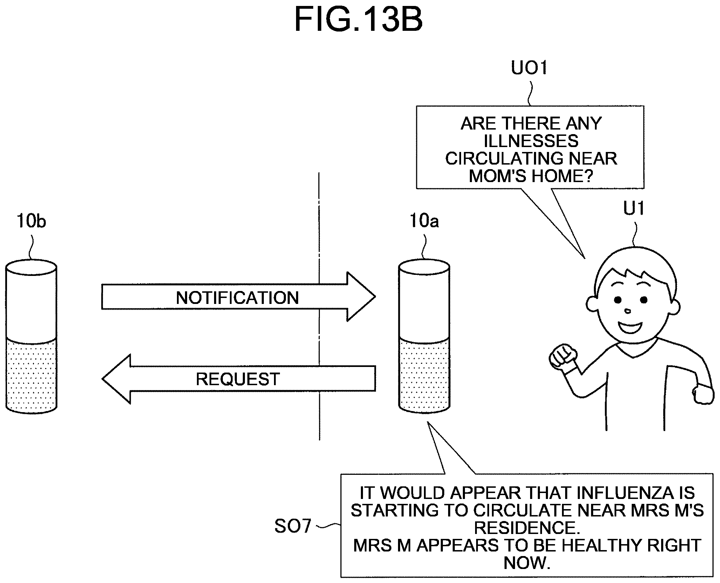

In addition, the information processing server 20 and the information processing terminal 10 according to the present embodiment may have a function for notifying the user of infectious disease epidemic information in a remote location, for example. FIGS. 13A and 13B are diagrams serving to illustrate control of epidemic information notification in a remote location.

For example, in the case of the example illustrated in FIG. 13A, the information processing server 20 is capable of recognizing, from sound information and image information or the like which are collected by an information processing terminal 10b which user U4 is using, that the number of coughs of a user U4 residing in a separate location from user U1 is increasing.

At such time, the information processing server 20 may control an information processing terminal 10a used by user U1 so that not only user U4 but also user U1, who is residing in a remote location, are notified of epidemic information about the place of residence of user U4. In the case of the example illustrated in FIG. 13A, the information processing server 20 uses a speech utterance SO6 to output, to the information processing terminal 10, the fact that user U4 is coughing more often and that the common cold is circulating in the vicinity of the place of residence of user U4.

In addition, FIG 13B illustrates an example of a case where the plurality of information processing terminals 10a and 10b each have the functions of the foregoing information processing server 20. At such time, the information processing terminal 10a is capable of issuing a request for epidemic information to the information processing terminal 10b installed in a remote location on the basis of an instruction using utterance UO1 of user U1, for example.

Furthermore, the information processing terminal 10b transmits, to the information processing terminal 10a, a notification pertaining to epidemic information about the vicinity of the installation location on the basis of the foregoing request and then, on the basis of the notification received by the information processing terminal 10a, uses a speech utterance SO7 to output, to user U1, infectious disease epidemic information about the vicinity of the installation location of the information processing terminal 10b. Note that the aforementioned notification of the epidemic information may be executed at regular intervals, for example, in addition to user instructions or may be executed a predetermined number of days before user U4 visits user U1, or the like.

Thus, by notifying a son and a daughter, or the like, of epidemic information relevant to their parents who are residing in a remote location, and so forth, for example, the information processing server 20 and information processing terminal 10 according to the present embodiment also make it possible to help both parties to live more safely and with more peace of mind.

Note that, although a case where the output control unit 240 according to the present embodiment controls the notification of epidemic information using speech utterances has been described as a principal example in the foregoing description, notification control according to the present embodiment is not limited to the example. The output control unit 240 according to the present embodiment may also control the notification of epidemic information accompanied by visual information.

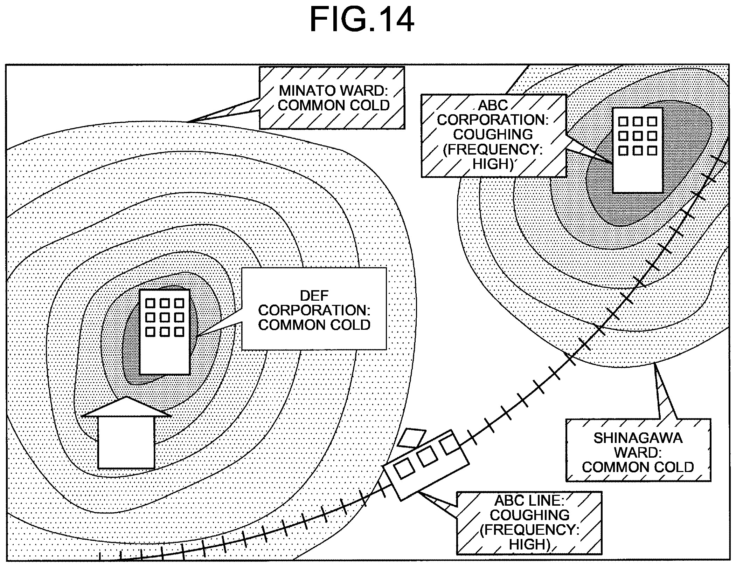

FIG. 14 is a diagram illustrating an example of epidemic information notification accompanied by visual information according to the present embodiment. In the case of the example illustrated in FIG. 14, the output control unit 240 causes the display unit 110 of the information processing terminal 10 to display the epidemic information extracted by the extraction unit 230 in association with map information.

More specifically, the output control unit 240 causes the display unit 110 to display the epidemic information collected on the basis of the profiles of users U1 and U2 described using FIG. 5 in association with map information such as the places of residence, work locations, workplaces, and routes to work of user U1 and user U2.

For example, the output control unit 240 uses a heat map and callouts to represent the epidemic condition of the common cold in Minato ward and Shinagawa ward and uses corresponding icons and callouts to represent infectious disease epidemic information for the workplaces and routes to work.

Thus, the output control unit 240 according to the present embodiment also makes it possible to control the notification of epidemic information accompanied by visual information and enables the user to grasp the epidemic information more intuitively.

Note that, in a case where the display of epidemic information relating to a plurality of users is controlled, the output control unit 240 may distinguish the epidemic information which is directly correlated with the user who is the notification target from the epidemic information relating to another user by highlighting the former epidemic information. In the case of the example illustrated in FIG. 14, because the notification target is user U1, the output control unit 240 uses hatching to highlight callouts pertaining to the epidemic information which has been collected on the basis of the profile of user U1.

The output control unit 240 may also control the notification of epidemic information for which visual information and speech utterances are used simultaneously. In this case, the output control unit 240 is also capable of performing control such as control in which only notification using visual information is to be secured from that point onward for epidemic information for which only notification using a speech utterance is complete, for example.

### <<1.6. Operation flow>>

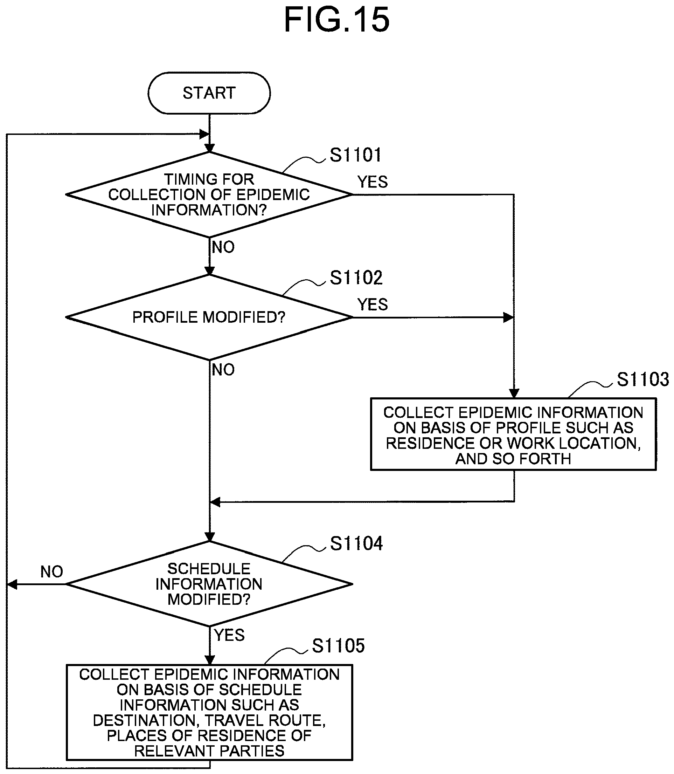

Next, the operation flow of the information processing server 20 according to the present embodiment will be described in detail. First, the flow of the collection of epidemic information by the information processing server 20 according to the present embodiment will be described. FIG. 15 is a flowchart illustrating the flow of epidemic information collection by the information processing server 20 according to the present embodiment.

Referring to FIG. 15, the collection unit 210 of the information processing server 20 first determines whether the timing for collecting the epidemic information has been reached (S1101). The collection unit 210 may make the foregoing determination by comparing the current time with a predetermined time which has been preconfigured, for example.

Here, when the timing for collecting the epidemic information has been reached (S1101: YES), the collection unit 210 collects epidemic information from the external device 30 or the internet on the basis of a profile such as the place of residence or the work location of the user (S1103).

When, on the other hand, the timing for collecting the epidemic information has not arrived (S1101: YES), the collection unit 210 then determines whether the profile has been modified (S1102).

Here, when the profile has been modified (S1102: YES), the collection unit 210 collects epidemic information on the basis of the modified profile (S1103).

When, on the other hand, the profile has not been modified (S1102: NO), the collection unit 210 then determines whether the schedule information has been modified (S1104).

Here, when the schedule information has not been modified (S1104: NO), the collection unit 210 returns to step S1101 and repeatedly executes this processing and subsequent processing.

When, on the other hand, the schedule information has been modified (S1104: YES), the collection unit 210 collects, on the basis of the modified schedule information, the epidemic information about the destination and travel route, and the place of residence of the relevant parties, and so forth (S1105).

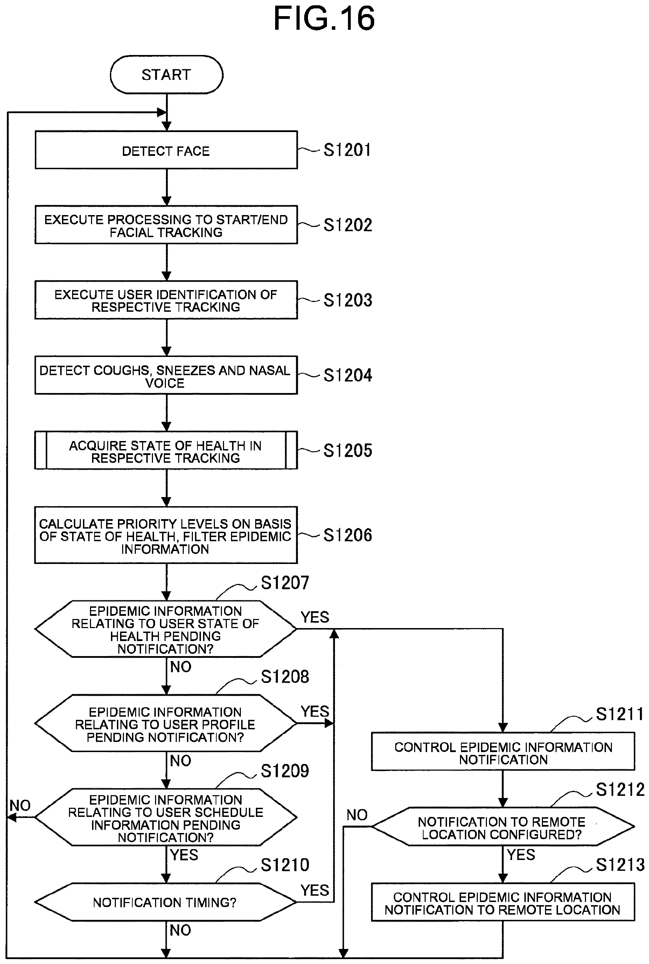

The flow of control of epidemic information notification by the information processing server 20 according to the present embodiment will be described next. FIG. 16 is a flowchart illustrating the flow of control of epidemic information notification by the information processing server 20 according to the present embodiment.

Referring to FIG. 16, the recognition unit 220 first detects the face of the user (S1201).

Thereafter, the recognition unit 220 executes start processing or end processing pertaining to facial tracking on the basis of the face detection result in step S1201 (S1202).

The recognition unit 220 then executes user identification processing in the respective tracking (S1203).

Furthermore the recognition unit 220 executes processing to detect coughing, sneezing, and a nasal voice, or the like (S1204).

The recognition unit 220 then acquires the state of health of the user in the respective tracking (S1205). Details of the state of health acquisition processing of step S1205 will be separately described subsequently.

Next, the extraction unit 230 calculates the priority level of each epidemic information item on the basis of the state of health acquired in step S1205 and executes filtering of the epidemic information on the basis of the priority levels (S1206). Specifically, the extraction unit 230 may execute filtering by calculating a high priority level for epidemic information in a location which is directly correlated with the user in a case where a problem with the state of health of the user has not been detected and by calculating a high priority level for epidemic information pertaining to an infectious disease which is similar to the symptoms of the user in a case where a problem with the state of health of the user has been detected.

Thereafter, the output control unit 240 determines, on the basis of the priority levels calculated in step S1206, whether epidemic information related to the state of health of the user is pending notification (S1207) and whether the epidemic information related to the profile of the user is pending notification (S1208).

Here, when there is epidemic information pending notification which relates to the state of health and profile (S1207: YES, or S1208: YES), the output control unit 240 controls the notification of the epidemic information for which notification has not yet been performed (S1211).

When, on the other hand, there is no epidemic information pending notification which relates to the profile (S1208: NO), and so forth, the output control unit 240 then determines whether the epidemic information related to the schedule information of the user is pending notification (S1209).

Here, when there is no epidemic information pending notification which relates to the schedule information of the user (S1209: NO), the information processing server 20 returns to step S1201 and repeatedly executes this processing and subsequent processing.

When, on the other hand, there is epidemic information pending notification which relates to the schedule information of the user (S1209: YES), the output control unit 240 then determines whether the notification timing for the epidemic information related to the schedule information has been reached (S1210).

Here, when the notification timing for the epidemic information related to the schedule information has not been reached (S1210: NO), the information processing server 20 returns to step S1201 and repeatedly executes this processing and subsequent processing.

When, on the other hand, the notification timing for the epidemic information related to the schedule information has been reached (S1210: YES), the output control unit 240 controls the notification of the epidemic information for which notification has not yet been performed (S1211).

Next, the output control unit 240 determines, for the epidemic information for which notification has been controlled in step S1211, whether there is a configuration for notification to a remote location (S1212).

Here, when there is no configuration for notification to a remote location (S1212: NO), the information processing server 20 returns to step S1201 and repeatedly executes this processing and subsequent processing.

When, on the other hand, there is a configuration for notification to a remote location (S1212: YES), the output control unit 240 controls the notification of epidemic information to the remote location on the basis of the configuration (S1213) and returns to step S1201.

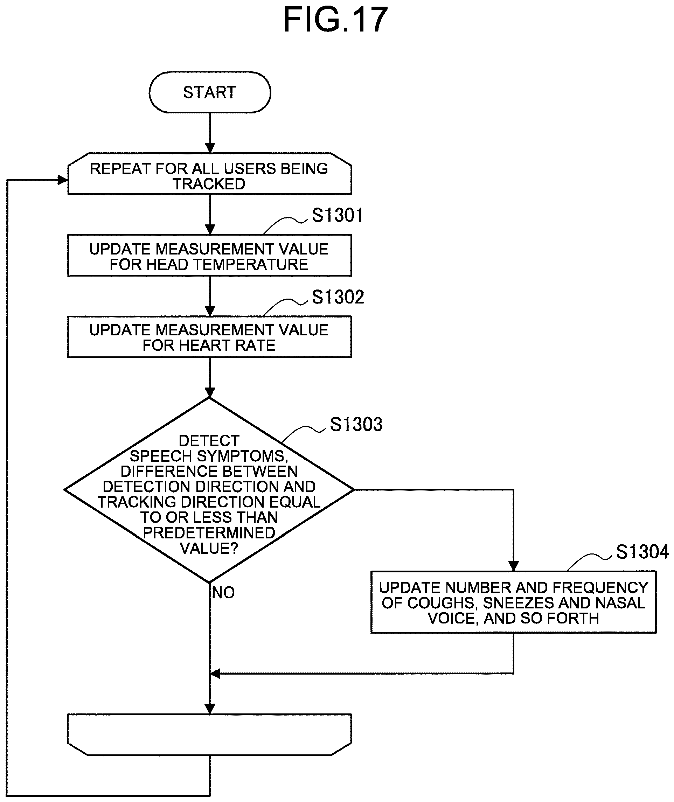

Next, the flow of the state of health acquisition in step S1205 of FIG. 16 will be described in detail. FIG. 17 is a flowchart illustrating the flow of state of health acquisition according to the present embodiment. Note that FIG. 17 illustrates, by way of an example, a case where the recognition unit 220 according to the present embodiment acquires, as examples of states of health, body temperature and heart rate measurements and the frequencies of coughing, sneezing and a nasal voice, or the like. However, the recognition unit 220 according to the present embodiment is not limited to this example and is capable of acquiring various states of health of the user.

Referring to FIG. 17, the recognition unit 220 measures the head temperature of the user being tracked and updates the stored measurement value (S1301).

In addition, the recognition unit 220 measures the heart rate of the user being tracked and updates the stored measurement value (S1302).

Next, the recognition unit 220 detects speech symptoms such as coughing, sneezing, and a nasal voice and determines whether the difference between the detection direction and the tracking direction is equal to or less than a predetermined value (S1303). That is, the recognition unit 220 determines whether or not the detected coughing, sneezing, or nasal voice, or the like, is produced by the user being tracked.

Here, when the difference between the speech symptoms detection direction and tracking direction is equal to or less than a predetermined value, the recognition unit 220 updates the number and frequency of coughs, sneezes, and nasal voice of the user being tracked (S1304).

The recognition unit 220 according to the present embodiment repeatedly executes the processing of steps S1301 to S1304 for all the users being tracked.

### <2. Hardware configuration example>

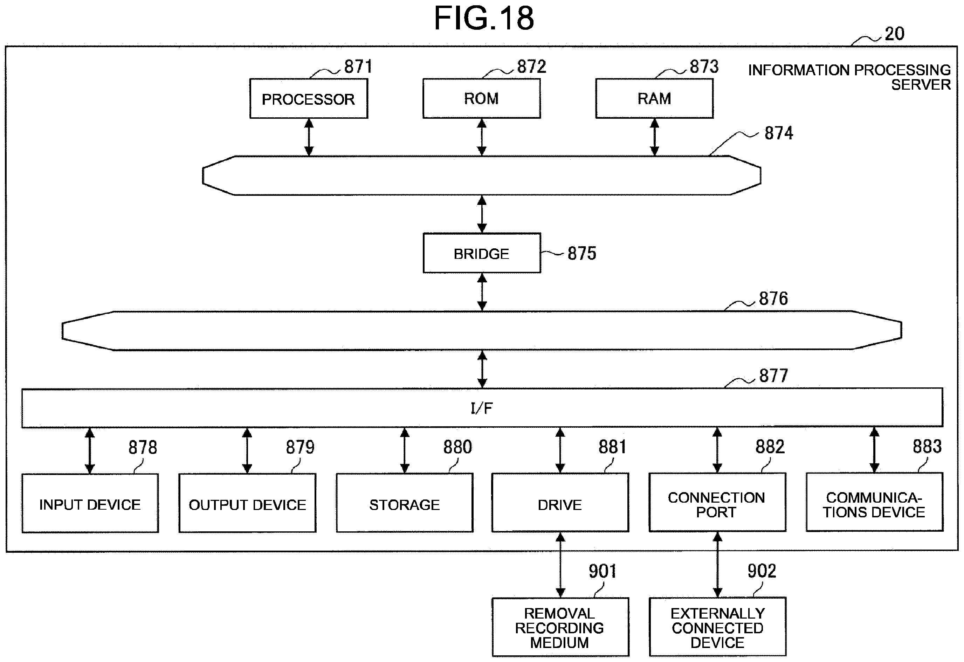

Next, a hardware configuration example of the information processing server 20 according to an embodiment of the present disclosure will be described. FIG. 18 is a block diagram illustrating a hardware configuration example of the information processing server 20 according to an embodiment of the present disclosure. Referring to FIG. 18, the information processing server 20 includes, for example, a processor 871, a ROM 872, a RAM 873, a host bus 874, a bridge 875, an external bus 876, an interface 877, an input device 878, an output device 879, storage 880, a drive 881, a connection port 882, and a communications device 883. Note that the hardware configuration illustrated here is an example, and some of the components may be omitted. In addition, components other than the components illustrated herein may be further included.

### (Processor 871)

For example, the processor 871 functions as an arithmetic processing device or a control device, and controls all or part of the operation of each component on the basis of various programs recorded in the ROM 872, the RAM 873, the storage 880 or a removable recording medium 901.

### (ROM 872 and RAM 873)

The ROM 872 is a means for storing a program read by the processor 871, data used for computation, or the like. The RAM 873 temporarily or permanently stores, for example, the program read by the processor 871, various parameters that change as appropriate when the program is executed, or the like.

### (Host bus 874, bridge 875, external bus 876, and interface 877)

The processor 871, the ROM 872, and the RAM 873 are mutually connected via, for example, the host bus 874, which is capable of high-speed data transmission. Meanwhile, the host bus 874 is connected to the external bus 876, which has a comparatively low data transmission rate, via the bridge 875, for example. In addition, the external bus 876 is connected to various components via the interface 877.

### (Input device 878)

As the input device 878, for example, a mouse, a keyboard, a touch panel, a button, a switch, a lever, or the like, is used. Further, a remote controller (hereinafter, remote control) capable of transmitting a control signal by using infrared rays or other radio waves may also be used as the input device 878. In addition, the input device 878 also includes a speech input device such as a microphone.

### (Output device 879)

The output device 879 is a device that is capable of visually or audibly notifying a user of acquired information such as, for example, a cathode ray tube (CRT), LCD, or organic EL display device, an audio output device such as a loudspeaker or headphones, a printer, a mobile phone, or a facsimile. In addition, the output device 879 according to the present disclosure includes various vibration devices that are capable of outputting tactile stimulation.

### (Storage 880)

The storage 880 is a device for storing various data. As the storage 880, for example, a magnetic storage device such as a hard disk drive (HDD), a semiconductor storage device, an optical storage device, a magneto-optical storage device, or the like, is used.

### (Drive 881)

The drive 881 is a device that reads information recorded on the removable recording medium 901 such as, for example, a magnetic disk, an optical disk, a magneto-optical disk, or a semiconductor memory, or writes information to the removable recording medium 901.

### (Removable recording medium 901)

The removable recording medium 901 is, for example, a DVD medium, a Blu-ray (registered trademark) medium, an HD DVD medium, or various semiconductor storage media, or the like. Naturally, the removable recording medium 901 may also be, for example, an IC card equipped with a noncontact IC chip, or an electronic device, or the like.

### (Connection port 882)

The connection port 882 is a port configured to connect an externally connected device 902 such as, for example, a universal serial bus (USB) port, an IEEE 1394 port, a small computer system interface (SCSI), an RS-232C port, or an optical audio terminal, or the like.

### (Externally connected device 902)

The externally connected device 902 is, for example, a printer, a portable music player, a digital camera, a digital video camera, or an IC recorder, or the like.

### (Communications device 883)

The communications device 883 is a communications device for connecting to a network and is, for example, a communications card for wired or wireless LAN, Bluetooth (registered trademark) or wireless USB (WUSB), or a router for optical communication, a router for asymmetric digital subscriber line (ADSL), or a modem for various communications, or the like.

### <3. Summary>

As described hereinabove, an information processing server 20 that implements the information processing method according to an embodiment of the present disclosure includes an output control unit 240 that controls notification of risk information pertaining to the health and safety of a user; and an extraction unit 230 that, on the basis of a situation of the user, dynamically filters the risk information, which is collected on the basis of a range of actions of the user. In addition, one characteristic of the output control unit 240 according to an embodiment of the present disclosure is that the user is notified of risk information that has been filtered by the extraction unit 230. This configuration makes it possible to realize the presentation of information relating to health and safety which is of a higher value to the user.

Preferred embodiments of the present disclosure have been described in detail above with reference to the accompanying drawings, but the technical scope of the present disclosure is not limited to or by such examples. It is obvious that a person ordinarily skilled in the technical field of the present disclosure could arrive at various modification examples or revised examples within the scope of the technological ideas disclosed in the claims, and it is naturally understood that such examples belong to the technical scope of the present disclosure.

Furthermore, the advantageous effects disclosed in the present specification are only descriptive or exemplary, and non-limiting. In other words, the technology according to the present disclosure affords, in addition to or instead of the foregoing advantageous effects, other advantageous effects which are obvious to a person skilled in the art from the disclosures of the present specification.

Furthermore, a program that enables hardware such as a CPU, a ROM, and a RAM which are built into a computer to afford the same functions as the configurations of the information processing server 20 may also be created, and a computer-readable recording medium whereon the program is recorded may also be provided.

Moreover, each of the steps pertaining to the processing by the information processing server 20 of the present specification need not necessarily be processed in chronological order as per the order described in the flowchart. For example, each of the steps pertaining to the processing by the information processing server 20 may be processed in a different order from the order described in the flowchart or may be processed in parallel.

Note that the following configurations also fall within the technical scope of the present disclosure.
(1) An information processing device, comprising:
   an output control unit that controls notification of risk information pertaining to health and safety of a user; and
   an extraction unit that, on a basis of a situation of the user, dynamically filters the risk information, which is collected on a basis of a range of actions of the user,
   wherein the output control unit notifies the user of the risk information which has been filtered by the extraction unit.
(2) The information processing device according to (1),
   wherein the extraction unit executes filtering of the risk information on a basis of a state of health of the user.
(3) The information processing device according to (2),
   wherein the extraction unit dynamically calculates priority levels of the respective risk information on the basis of the state of health of the user and executes filtering of the risk information on a basis of the priority levels.
(4) The information processing device according to (3),
   wherein the extraction unit calculates as higher the priority level of the risk information which is highly correlated with the state of health of the user, and
   the output control unit preferentially notifies the user of the risk information having the higher priority level.
(5) The information processing device according to any one of (1) to (4),
   wherein the risk information includes infectious disease epidemic information, and
   the output control unit notifies the user of the epidemic information filtered by the extraction unit.
(6) The information processing device according to (5),
   wherein the extraction unit dynamically filters, on a basis of a state of health of the user, the epidemic information collected on a basis of a range of actions of relevant parties who may be a factor in contracting an infectious disease.
(7) The information processing device according to (6),
   wherein, when a problem with the state of health of the user has not been detected, the extraction unit extracts the epidemic information collected on the basis of the range of actions of the user, and
   the output control unit notifies the user of the epidemic information pertaining to the range of actions of the user which has been extracted by the extraction unit.
(8) The information processing device according to (6) or (7),
   wherein, when a problem with the state of health of the user has been detected, the extraction unit extracts, on the basis of the state of health of the user, the epidemic information collected on the basis of the range of actions of the user and the relevant parties, and
   the output control unit notifies the user of the epidemic information which has been extracted by the extraction unit.
(9) The information processing device according to (8),
   wherein, when a problem with the state of health of the user has been detected, the extraction unit extracts, from among the epidemic information which has been collected on the basis of the range of actions of the user and the relevant parties, epidemic information which is highly correlated with the state of health of the user.
(10) The information processing device according to (9),
   wherein the extraction unit extracts the epidemic information pertaining to the infectious disease for which symptoms are similar to the state of health of the user.
(11) The information processing device according to any one of (6) to (10),
   wherein the output control unit notifies the user of information relating to preventing or dealing with the infectious disease which corresponds to the epidemic information extracted by the extraction unit.
(12) The information processing device according to any one of (6) to (11),
   wherein the range of actions of the user or the relevant parties is acquired on a basis of at least any of a place of residence, a workplace or a place of study, and a route to work or a route to a place of study.
(13) The information processing device according to (12),
   wherein the relevant parties include at least a family member of the user.
(14) The information processing device according to any one of (6) to (13),
   wherein the range of actions of the user is acquired on a basis of schedule information of the user, and
   the schedule information includes information relating to at least any of a destination, a travel route, or the relevant parties.
(15) The information processing device according to (14),
   wherein the relevant parties include at least either a companion or a visitor.
(16) The information processing device according to any one of (6) to (15),
   wherein, when the user has been recognized, the output control unit notifies the user of the epidemic information.
(17) The information processing device according to any one of (6) to (16),
   wherein the output control unit notifies a guardian of the user of the epidemic information.
(18) The information processing device according to any one of (6) to (17),
   wherein the output control unit displays the epidemic information in association with map information.
(19) The information processing device according to any one of (1) to (18), further comprising:
   a collection unit that collects the risk information on the basis of the range of actions of the user.
(20) An information processing method, by a processor, comprising:
   controlling notification of risk information pertaining to health and safety of a user; and
   dynamically filtering, on a basis of a situation of the user, the risk information, which is collected on a basis of a range of actions of the user,
   wherein the controlling further includes notifying the user of the filtered risk information.

### Reference Signs List

- 10: INFORMATION PROCESSING TERMINAL
- 20: INFORMATION PROCESSING SERVER
- 210: COLLECTION UNIT
- 220: RECOGNITION UNIT
- 230: EXTRACTION UNIT
- 240: OUTPUT CONTROL UNIT
- 250: RISK INFORMATION DB
- 260: USER DB
- 270: TERMINAL COMMUNICATIONS UNIT
- 30: EXTERNAL DEVICE

## Claims

1. An information processing device, comprising:
an output control unit that controls notification of risk information pertaining to health and safety of a user; and
an extraction unit that, on a basis of a situation of the user, dynamically filters the risk information, which is collected on a basis of a range of actions of the user,
wherein the output control unit notifies the user of the risk information which has been filtered by the extraction unit.

2. The information processing device according to claim 1,
wherein the extraction unit executes filtering of the risk information on a basis of a state of health of the user.

3. The information processing device according to claim 2,
wherein the extraction unit dynamically calculates priority levels of the respective risk information on the basis of the state of health of the user and executes filtering of the risk information on a basis of the priority levels.

4. The information processing device according to claim 3,
wherein the extraction unit calculates as higher the priority level of the risk information which is highly correlated with the state of health of the user, and
the output control unit preferentially notifies the user of the risk information having the higher priority level.

5. The information processing device according to claim 1,
wherein the risk information includes infectious disease epidemic information, and
the output control unit notifies the user of the epidemic information filtered by the extraction unit.

6. The information processing device according to claim 5,
wherein the extraction unit dynamically filters, on a basis of a state of health of the user, the epidemic information collected on a basis of a range of actions of relevant parties who may be a factor in contracting an infectious disease.

7. The information processing device according to claim 6,
wherein, when a problem with the state of health of the user has not been detected, the extraction unit extracts the epidemic information collected on the basis of the range of actions of the user, and
the output control unit notifies the user of the epidemic information pertaining to the range of actions of the user which has been extracted by the extraction unit.

8. The information processing device according to claim 6,
wherein, when a problem with the state of health of the user has been detected, the extraction unit extracts, on the basis of the state of health of the user, the epidemic information collected on the basis of the range of actions of the user and the relevant parties, and
the output control unit notifies the user of the epidemic information which has been extracted by the extraction unit.

9. The information processing device according to claim 8,
wherein, when a problem with the state of health of the user has been detected, the extraction unit extracts, from among the epidemic information which has been collected on the basis of the range of actions of the user and the relevant parties, epidemic information which is highly correlated with the state of health of the user.

10. The information processing device according to claim 9,
wherein the extraction unit extracts the epidemic information pertaining to the infectious disease for which symptoms are similar to the state of health of the user.

11. The information processing device according to claim 6,
wherein the output control unit notifies the user of information relating to preventing or dealing with the infectious disease which corresponds to the epidemic information extracted by the extraction unit.

12. The information processing device according to claim 6,
wherein the range of actions of the user or the relevant parties is acquired on a basis of at least any of a place of residence, a workplace or a place of study, and a route to work or a route to a place of study.

13. The information processing device according to claim 12,
wherein the relevant parties include at least a family member of the user.

14. The information processing device according to claim 6,
wherein the range of actions of the user is acquired on a basis of schedule information of the user, and
the schedule information includes information relating to at least any of a destination, a travel route, or the relevant parties.

15. The information processing device according to claim 14,
wherein the relevant parties include at least either a companion or a visitor.

16. The information processing device according to claim 6,
wherein, when the user has been recognized, the output control unit notifies the user of the epidemic information.

17. The information processing device according to claim 6,
wherein the output control unit notifies a guardian of the user of the epidemic information.

18. The information processing device according to claim 6,
wherein the output control unit displays the epidemic information in association with map information.

19. The information processing device according to claim 1, further comprising:
a collection unit that collects the risk information on the basis of the range of actions of the user.

20. An information processing method, by a processor, comprising:
controlling notification of risk information pertaining to health and safety of a user; and
dynamically filtering, on a basis of a situation of the user, the risk information, which is collected on a basis of a range of actions of the user,
wherein the controlling further includes notifying the user of the filtered risk information.
